# EUROPÄISCHE PATENTSCHRIFT

(11) **EP 0 722 933 B1**
(45) Veröffentlichungstag und Bekanntmachung des Hinweises auf die Patenterteilung: **09.10.2002**
(21) Anmeldenummer: 95119458.8
(22) Anmeldetag: 11.12.1995
(51) Int. Cl.: C07D 213/61

(54) **Verfahren zur Herstellung von Chlormethylpyridin Derivaten und Acylaminomethylpyridine als Zwischenprodukte**
Process for the preparation of chloromethylpyridine derivatives and acylaminomethyl pyridines as intermediates
Procédé pour le préparation de dérivés de chlorométhylpyridine et acylaminométhylpyridines comme produits intermédiaires

(30) Priorität: 23.12.1994 DE 4446338
(43) Veröffentlichungstag der Anmeldung: 24.07.1996
(73) Patentinhaber: BAYER AG, 51368 Leverkusen (DE)
(72) Erfinder: Lantzsch, Reinhrad, Dr., D-42115 Wuppertal (DE)

(56) Entgegenhaltungen:
- EP-A- 0 556 684
- EP-A- 0 691 331
- WO-A-95/18122
- CHEMICAL ABSTRACTS, vol. 116, no. 7, 17.Februar 1992 Columbus, Ohio, US; abstract no. 59366r, KAKU S. ET AL. 'Preparation of chlorine-containing heterocyclic group-substituted methylamines' Seite 862; Spalte 2; & JP-A-03 223 252 (NIPPON SODA CO., LTD.) 2.Oktober 1991
- JUSTUS LIEBIGS ANNALEN DER CHEMIE, Bd. 449, Nr. 2/3, 30.September 1926 Seiten 249-277, VON BRAUN J. ET AL. 'Haftfestigkeit organischer Reste. V'
- JOURNAL OF THE AMERICAN CHEMICAL SOCIETY, Bd. 84, Nr. 5, 13.März 1962 Seiten 769-774, XP 000561172 VAUGHAN W.R. & CARLSON R.D. 'The von Braun reaction. I. Scope and limitations'
- BIERON J.F. & DINAN F.J. 'Rearrangement and elimination of the amido group' , IN: "THE CHEMISTRY OF AMIDES" (ZABICKY J. ED.) 1970; PP.245-88 , INTERSCIENCE PUBLISHERS, TORONTO XP 000567845 * Seite 277 - Seite 279 *
- HETEROCYCLES, Bd. 15, Nr. 1, 1981 Seiten 369-371, TACHIKAWA R. ET AL. 'Synthesis of imidazolopyridine derivatives by an unusual reaction of N-benzylpicolinamides with phosphorus pentachloride'
- JOURNAL OF THE CHEMICAL SOCIETY, PERKIN TRANSACTIONS 1, 1981 Seiten 1537-1543, XP 000561173 METH-COHN O. ET AL. 'A versatile new synthesis of quinolines and related fused pyridines. Part 8. Conversion of anilides into 3-sibstituted quinolines and into quinoxalines'
- BOEHME H. & VIEHE H.G.: 'Iminium salts in organic chemistry, part 2' ADVANCES IN ORGANIC CHEMISTRY: METHODS AND RESULTS 1976, JOHN WILEY & SONS, NEW YORK, Seite 67

## Beschreibung

Die Erfindung betrifft ein neues Verfahren zur Herstellung von Chlormethylpyridinen sowie neue Zwischenprodukte zu ihrer Herstellung.

Es ist bekannt, dass Chlormethylpyridine durch Umsetzung von Hydroxymethylpyridinen mit Thionylchlorid erhalten werden können (vgl. J. Heterocycl. Chem. 16 (1979), 333-337; vgl. auch EP-A 373 464).

Weiter ist bekannt, dass man Chlormethylpyridine erhält, wenn man Methylpyridine in Gegenwart von Säureakzeptoren oder Radikalbildnern und in Gegenwart von inerten Verdünnungsmitteln bei Temperaturen zwischen 0°C und 100°C chloriert (vgl. EP-A 260 485, vgl. auch EPA 458 109).

Ferner ist bekannt, dass auch Alkoxymethylpyridine durch Umsetzung mit geeigneten Chlorierungsmitteln in Chlormethylpyridine umgewandelt werden können (vgl. EP-A 393 453).

Außerdem ist bekannt, dass Chlormethylpyridine durch Diazotierung in Gegenwart von beispielsweise Chlorwasserstoff aus entsprechenden Aminomethylpyridinen erhalten werden können (vgl. JP 05 178 835).

Bei diesen bekannten Synthesemethoden sind jedoch die erzielbaren Ausbeuten und die Qualitäten der Produkte nicht immer zufriedenstellend.

Es wurde nun gefunden, dass man Chlormethylpyridine der allgemeinen Formel (I) 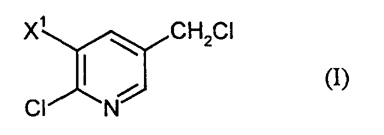 in welcher
- X¹: für Wasserstoff, Chlor oder Methyl steht,
in guten Ausbeuten und in hoher Reinheit erhält,
wenn man
Pyridin-Derivate der allgemeinen Formel (II) 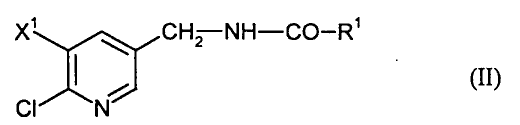 in welcher
- R¹: für Wasserstoff, für geradkettiges oder verzweigtes Alkyl mit 1 bis 4 Kohlenstoffatomen, sowie für gegebenenfalls einfach bis dreifach gleich oder verschieden substituiertes Phenyl, wobei als Substituenten genannt seien: Halogen, geradkettiges oder verzweigtes Alkyl mit 1 bis 4 Kohlenstoffatomen, geradkettiges oder verzweigtes Alkoxy und Alkylthio mit jeweils 1 bis 2 Kohlenstoffatomen, geradkettiges oder verzweigtes Halogenalkyl, Halogenalkoxy und Halogenalkylthio mit jeweils 1 bis 2 Kohlenstoffatomen und 1 bis 5 gleichen oder verschiedenen Halogenatomen, sowie gegebenenfalls durch Chlor und/oder Methyl substituiertes Phenyl steht,
- X¹: die oben angegebene Bedeutung hat,
mittels "Vilsmeier Reagenz", d.h. mit einem Formamid-Derivat der allgemeinen Formel (III) 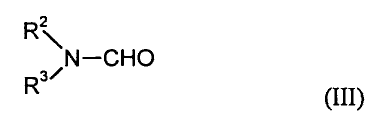 in welcher
- R² und R³: gleich oder verschieden sind und für geradkettiges oder verzweigtes Alkyl mit 1 bis 6 Kohlenstoffatomen, ferner für Cycloalkyl mit 3 bis 6 Kohlenstoffatomen, sowie gemeinsam für Alkandiyl mit 2 bis 6 Kohlenstoffatomen stehen,
sowie mit einem Chlorierungsmittel, gegebenenfalls in Gegenwart eines Verdünnungsmittels bei Temperaturen zwischen 20 und 120°C umsetzt und anschließend auf übliche Weise aufarbeitet.

Überraschenderweise können nach dem erfindungsgemäßen Verfahren Chlormethylpyridine der Formel (I) in sehr guten Ausbeuten und in hoher Reinheit erhalten werden, obwohl nach dem Stand der Technik zum einen eine Formylierung der NH-Gruppe zu erwarten war (vgl. z.B. J.C.S. Perkin I, S. 1537-1543 (1981)); zum anderen die Umsetzung gemäß der "von Braun Reaction" (vgl. JACS 84, 769-774 (1962)) mit Phosphorpentachlorid bzw. Sulfonylchlorid als Chlorierungsmittel nicht gelingt.

Verwendet man beispielsweise 2,3-Dichlor-5-acetaminomethylpyridin als Ausgangsstoff sowie Dimethylformamid und Oxalylchlorid als "Vilsmeier Reagenz", so kann der Reaktionsablauf beim erfindungemäßen Verfahren durch das folgende Formelschema skizziert werden:

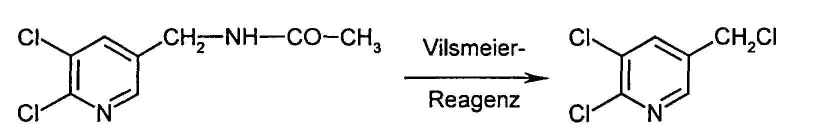

Die beim erfindungsgemäßen Verfahren als Ausgangsstoffe zu verwendenden Pyridin-Derivate sind durch die Formel (II) allgemein definiert. In der Formel (II) steht X¹ für Wasserstoff, Chlor oder Methyl. R¹ steht vorzugsweise für Wasserstoff, für Methyl, Ethyl, sowie für gegebenenfalls einfach bis dreifach, gleich oder verschieden substituiertes Phenyl, wobei als Substituenten genannt seien: Fluor, Chlor, Methyl, Ethyl, Isopropyl, Methoxy, Methylthio, Trifluormethyl, Trifluormethoxy oder Trifluormethylthio, sowie gegebenenfalls durch Chlor und/oder Methyl substituiertes Phenyl.

Die Pyridin-Derivate der Formel (II) sind teilweise bekannt (vgl. EP-A 0 556 684, JP-A 03 233 252 und EP-A 0 691 331). Pyridin-Derivate der allgemeinen Formel (IIa) 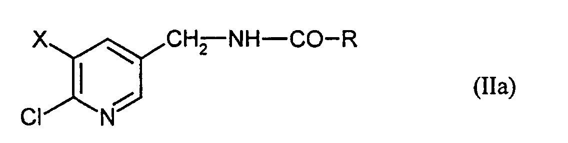 in welcher
- X: für Wasserstoff, Chlor oder Methyl steht,
- R: für geradkettiges oder verzweigtes Alkyl mit 1 bis 4 Kohlenstoffatomen, wie insbesondere Methyl und Ethyl steht; oder für gegebenenfalls einfach bis dreifach, gleich oder verschieden substituiertes Phenyl steht, wobei als Substituenten genannt seien: Halogen, wie insbesondere Fluor oder Chlor; geradkettiges oder verzweigtes Alkyl mit 1 bis 4 Kohlenstoffatomen, wie insbesondere Methyl und Ethyl sowie für gegebenenfalls einfach bis dreifach gleich oder verschieden substituiertes Phenyl, wobei als Substituenten genannt seien: Halogen, wie insbesondere Fluor oder Chlor; geradkettiges oder verzweigtes Alkyl mit 1 bis 4 Kohlenstoffatomen, wie insbesondere Methyl, Ethyl oder Isopropyl; geradkettiges oder verzweigtes Alkoxy und Alkylthio mit jeweils 1 bis 2 Kohlenstoffatomen, wie insbesondere Methoxy oder Methylthio; geradkettiges oder verzweigtes Halogenalkyl, Halogenalkoxy und Halogenalkylthio mit jeweils 1 bis 2 Kohlenstoffatomen und 1 bis 5 gleichen oder verschiedenen Halogenatomen, wie Fluor- und Chloratome, wie insbesondere Trifluormethyl, Trifluormethoxy oder Trifluormethylthio; sowie gegebenenfalls durch Chlor und/oder Methyl substituiertes Phenyl,
werden erhalten, indem man Aminomethylpyridine der allgemeinen Formel (IV) 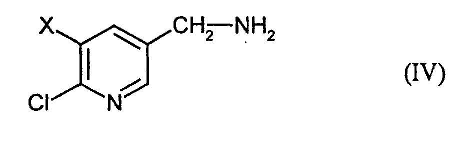 in welcher
- X: die oben angegebene Bedeutung hat,
mit Säurechloriden der allgemeinen Formel (V)

Cl―CO―R (V)

in welcher
- R: die oben angegebene Bedeutung hat,
in Gegenwart eines Reaktionshilfsmittels und gegebenenfalls in Gegenwart eines Verdünnungsmittels umsetzt.

Als Verdünnungsmittel kommen bei der Durchführung des Verfahrens zur Herstellung der Pyridin-Derivate der Formel (IIa) alle für derartige Umsetzungen üblichen, inerten organischen Solventien in Betracht. Vorzugsweise verwendbar sind Ester, wie Essigsäuremethylester oder Essigsäureethylester, ferner Ether, wie Diethylether, Methyl-tert.-butyl-ether, Ethylenglykol-dimethylether, Tetrahydrofuran und Dioxan, ferner Nitrile, wie Acetonitril, und außerdem gegebenenfalls halogenierte aliphatische, cycloaliphatische und aromatische Kohlenwasserstoffe, wie Methylenchlorid, Chloroform, Tetrachlorkohlenstoff, Hexan, Cyclohexan, Benzol, Toluol, Xylol und Chlorbenzol.

Als Reaktionshilfsmittel kommen bei der Durchführung des Verfahrens zur Herstellung der Pyridin-Derivate der Formel (IIa) alle üblichen anorganischen oder organischen Basen in Frage. Vorzugsweise verwendbar sind Alkalimetall- oder Erdalkalimetallhydroxide, -amide, -alkoholate, -carbonate und -hydrogencarbonate, wie Natriumhydroxid, Kaliumhydroxid, Natriumamid, Natriummethylat, Natriumethylat, Kaliumtert.-butylat, Natriumcarbonat, Kaliumcarbonat, Calciumcarbonat, Kaliumhydrogencarbonat und Natriumhydrogencarbonat, und weiterhin tertiäre Amine, wie Trimethylamin, Triethylamin, Tributylamin, N,N-Dimethylanilin, Pyridin, N-Methylpiperidin, N,N-Dimethylaminopyridin, Diazabicyclooctan (DABCO), Diazabicyclononen (DBN) oder Diazabicycloundecen (DBU).

Die Reaktionstemperaturen können bei der Durchführung des Verfahrens zur Herstellung der Pyridin-Derivate der Formel (IIa) innerhalb eines größeren Bereiches variiert werden. Im allgemeinen arbeitet man bei Temperaturen zwischen 0 und 120°C, vorzugsweise zwischen 10 und 100°C.

Bei der Durchführung des Verfahrens zur Herstellung der Pyridin-Derivate der Formel (IIa) setzt man pro Mol Aminomethylpyridin der Formel (IV) im allgemeinen 1,0 bis 3,0 Mol, vorzugsweise 1,0 bis 1,5 Mol an Säurechlorid der Formel (V) sowie 1,0 bis 3,0 Mol, vorzugsweise 1,0 bis 1,5 Mol an Reaktionshilfsmittel ein. Die Reaktionsdurchführung, Aufarbeitung und Isolierung der Reaktionsprodukte erfolgt nach allgemein üblichen Methoden.

Die Aminomethylpyridine der Formel (IV) sind bekannt (vgl. z.B. US 5 300 650, EP-A 0 579 970) bzw. können sie nach den dort beschriebenen Verfahren in bekannter Art und Weise erhalten werden.

Die Säurechloride der Formel (V) sind allgemein bekannte Verbindungen der organischen Chemie.

Pyridin-Derivate der Formel (IIa) können auch erhalten werden, indem man Aminomethylpyridine der Formel (IV) in allgemein bekannter Art und Weise mit entsprechenden Anhydriden umsetzt.

Die außerdem beim erfindungsgemäßen Verfahren als Ausgangsstoffe zu verwendenden Formamid-Derivate sind durch die Formel (III) allgemein definiert. In der Formel (III) sind R² und R³ gleich oder verschieden und stehen vorzugsweise für Methyl, Ethyl, n- oder i-Propyl, n-, i-, s- oder t-Butyl; Cyclopentyl oder Cyclohexyl; sowie gemeinsam für Butan-1,4-diyl oder Pentan-1,5-diyl.

Als Beispiele für die Formamid-Derivate der Formel (III) seien genannt:
N,N-Dimethyl-formamid, N,N-Diethyl-formamid, N,N-Dipropyl-formamid, N,N-Dibutyl-formamid, N-Cyclohexyl-N-methyl-formamid, N,N-Dicyclohexyl-formamid.

Als besonders bevorzugte Formamid-Derivate seien N,N-Dimethyl-formamid und N,N-Dibutyl-formamid genannt.

Die Formamid-Derivate der Formel (III) sind bekannte organische Synthesechemikalien.

Das erfindungsgemäße Verfahren wird unter Verwendung eines Chlorierungsmittels durchgeführt. Es können hierbei die üblichen Chlorierungsmittel verwendet werden, beispielsweise Phosphorylchlorid (Phosphoroxychlorid), Phosphor(V)chlorid, Phosgen, Oxalylchlorid, Thionylchlorid, Perchlorbutansäurechlorid, Dichlorbenzodioxol, N,N-Dimethyl-chlormethylimmoniumchlorid oder N,N-Diethyl-chlormethylimmoniumchlorid.

Phosgen wird als Chlorierungsmittel beim erfindungsgemäßen Verfahren besonders bevorzugt.

Als Verdünnungsmittel zur Durchführung des erfindungsgemäßen Verfahrens kommen die üblichen organischen Lösungsmittel in Betracht. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform, Tetrachlormethan; Ether, wie Diethylether, Diisopropylether, Dioxan, Tetrahydrofuran oder Ethylenglykoldimethyl- oder -diethylether; Nitrile, wie Acetonitril, Propionitril oder Butyronitril; Amide, wie N,N-Dimethylformamid, N,N-Dimethylacetamid, N-Methylformanilid, N-Methyl-pyrrolidon oder Hexamethylphosphorsäuretriamid; Sulfoxide wie Dimethylsulfoxid, sowie Sulfone, wie Tetramethylensulfon.

Als besonders bevorzugte Verdünnungsmittel seien Chlorbenzol, Acetonitril und Butyronitril genannt.

Die Reaktionstemperaturen können bei der Durchführung des erfindungsgemäßen Verfahrens in einem größeren Bereich variiert werden. Im allgemeinen arbeitet man bei Temperaturen zwischen -30°C und +150°C, vorzugsweise bei Temperaturen zwischen -10°C und +120°C, wobei in der Anfangsphase vorzugsweise zwischen -10°C und +40°C und anschließend zwischen +20°C und 120°C gearbeitet wird. Man kann jedoch auch direkt bei erhöhter Temperatur arbeiten und das "Vilsmeier Reagenz" in situ erzeugen.

Das erfindungsgemäße Verfahren wird im allgemeinen unter Normaldruck durchgerührt. Es ist jedoch auch möglich, unter erhöhtem oder vermindertem Druck - im allgemeinen zwischen 0,1 bar und 10 bar - zu arbeiten.

Zur Durchführung des erfindungsgemäßen Verfahrens setzt man pro Mol Pyridin-Derivat der Formel (II) im allgemeinen zwischen 1 und 10 Mol, vorzugsweise zwischen 1,5 bis 5,0 Mol, insbesondere zwischen 2,0 und 4,0 Mol Chlorierungsmittel und zwischen 1 und 10 Mol, vorzugsweise zwischen 2,0 und 4,0 Mol Formamid-Derivat der Formel (III) ein.

Die Reaktionskomponenten können bei der Durchführung des erfindungsgemäßen Verfahrens in beliebiger Reihenfolge mit einander umgesetzt werden.

In einer bevorzugten Ausführungsform des erfindungsgemäßen Verfahrens werden zunächst das Formamid-Derivat der Formel (III) und das Chlorierungsmittel in einem Verdünnungsmittel vorgelegt und das Pyridin-Derivat der Formel (II) wird dann bei Temperaturen zwischen -10°C und +50°C zudosiert. Man führt danach die Umsetzung durch ein- oder mehrstündiges Rühren bei erhöhter Temperatur zu Ende.

Die Aufarbeitung kann auf übliche Weise durchgeführt werden. Beispielsweise wird mit Wasser auf das zwei- bis dreifache Volumen verdünnt. Die wässrige Phase wird druch Zugabe einer Base, wie z.B. Natronlauge, auf einen pH-Wert zwischen 2 und 7 eingestellt und danach wird mittels eines Lösungsmittels extrahiert und dieses dann unter vermindertem Druck sorgfältig abdestilliert. Das als Rückstand verbleibende Rohprodukt kann auf übliche Weise weiter gereinigt werden; es kann aber auch als solches zu weiteren Umsetzungen verwendet werden. Eine Isolierung durch Wasserdampfdestillation ist ebenfalls möglich.

Die nach dem erfindungegemäßen Verfahren herzustellenden Chlormethylpyridine der Formel (I) können als Zwischenprodukte zur Herstellung von biologisch aktiven Verbindungen; beispielsweise von Insekten verwendet werden (vgl. EP-A 163 855 und EP-A 192 060).

### Herstellungsbeispiele

### Beispiel 1

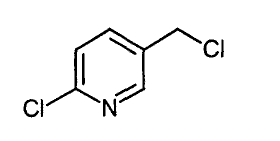

4,95 g (0,02 Mol) 2-Chlor-5-benzoylaminopyridin (vgl. Beispiel IIa-2) und 4,4 g (0,06 Mol) Dimethylformamid werden in 20 ml Butyronitril gelöst. Dann werden ohne Kühlung 6,6 g Phosgen eingeleitet. Anschließend erhitzt man 5 Stunden auf 50°C und eine Stunde auf 115°C.

Nach dem Abkühlen gießt man auf Eiswasser und extrahiert dreimal mit Methylenchlorid. Die vereinigten organischen Phasen werden getrocknet und eingeengt. Das zurückbleibende schwarze Öl wird durch Destillation im Kugelrohr (Ölpumpenvakuum) gereinigt (Manteltemperatur 70-80°C).

Man erhält 1,8 g (55,6% der Theorie) 2-Chlor-5-chlormethyl-pyridin vom Schmelzpunkt 35-36°C.

### Beispiel 2

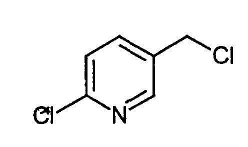

37 g (0,2 Mol) 2-Chlor-5-acetaminomethylpyridin (vgl. Beispiel IIa-1) und 44 g (0,06 Mol) Dimethylformamid werden in 200 ml Acetonitril gelöst. Bei 15°C werden 76 g (0,06 Mol) Oxalylchlorid zugetropft und anschließend auf 80°C erhitzt. Man rührt 18 Stunden bei dieser Temperatur, kühlt ab und gießt auf Eiswasser. Nach dreimaligem Extrahieren mit Methylenchlorid werden die organischen Phasen getrocknet und eingeengt. Man erhält 24 g eines schwarzen Öls, das durch Destillation gereinigt wird.

Man erhält 23,4 g (72,2% der Theorie) 2-Chlor-5-chlormethylpyridin vom Schmelzpunkt 35-36°C.

### Beispiel 3

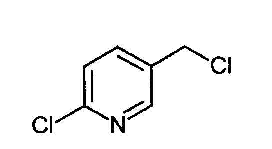

Der Ansatz wird analog Beispiel 2 durchgeführt, jedoch werden statt 76 g Oxalylchlorid werden 60 g Phosgen eingesetzt.

Man erhält 24,2 g (74,7% der Theorie) 2-Chlor-5-chlormethylpyridin vom Schmelzpunkt 35-36°C.

### Herstellung der Vorprodukte der Formel (IIa)

### Beispiel (IIa-1)

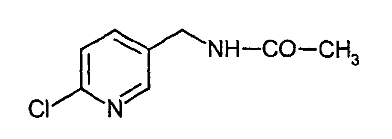

28,5 g (0,2 Mol) 2-Chlor-5-aminomethylpyridin und 22,2 g (0,22 Mol) Triethylamin werden in 300 ml Essigester gelöst. Bei 20°C werden dann unter Kühlung 16,5 g (0,21 Mol) Acetylchlorid zugetropft. Man rührt über Nacht nach, wäscht zweimal mit Wasser, trocknet und engt ein.

Man erhält 30,5 g (83% der Theorie) 2-Chlor-5-acetaminomethylpyridin als hellgelbe Kristalle vom Schmelzpunkt 76-77°C.

### Beispiel (IIa-2)

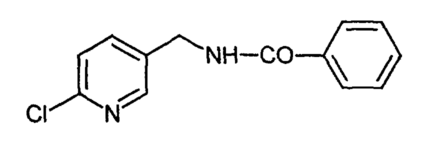

14,25 g (0,1 Mol) 2-Chlor-5-aminomethylpyridin und 11,1 g (0,11 Mol) Triethylamin werden in 150 ml Methylenchlorid gelöst. Dann werden bei ca. 20°C 14,8 g (0,105 Mol) Benzoylchlorid zugetropft und über Nacht bei Raumtemperatur nachgerührt. Man wäscht dreimal mit Wasser, trocknet die organische Phase und engt ein.

Man erhält 24,6 g (99,8% der Theorie) 2-Chlor-5-benzoylaminopyridin als weiße Kristalle vom Schmelzpunkt 118-120°C.

## Patentansprüche

1. Verfahren zur Herstellung von Chlormethylpyridinen der allgemeinen Formel (I) 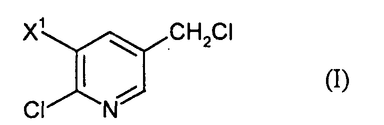 in welcher
X¹ für Wasserstoff, Chlor oder Methyl steht,
**dadurch gekennzeichnet, dass** man
Pyridin-Derivate der allgemeinen Formel (II) 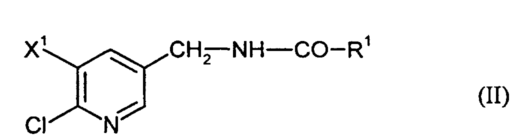 in welcher
R¹ für Wasserstoff, für geradkettiges oder verzweigtes Alkyl mit 1 bis 4 Kohlenstoffatomen, sowie für gegebenenfalls einfach bis dreifach, gleich oder verschieden substituiertes Phenyl, wobei als Substituenten genannt seien: Halogen, geradkettiges oder verzweigtes Alkyl mit 1 bis 4 Kohlenstoffatomen, geradkettiges oder verzweigtes Alkoxy und Alkylthio mit jeweils 1 bis 2 Kohlenstoffatomen, geradkettiges oder verzweigtes Halogenalkyl, Halogenalkoxy und Halogenalkylthio mit jeweils 1 bis 2 Kohlenstoffatomen und 1 bis 5 gleichen oder verschiedenen Halogenatomen, sowie gegebenenfalls durch Chlor und/oder Methyl substituiertes Phenyl steht,
X¹ die oben angegebene Bedeutung hat,
mittels einem Formamid-Derivat der allgemeinen Formel (III) 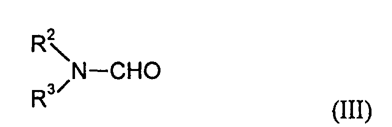 in welcher
R² und R³ gleich oder verschieden sind und für geradkettiges oder verzweigtes Alkyl mit 1 bis 6 Kohlenstoffatomen, ferner für Cycloalkyl mit 3 bis 6 Kohlenstoffatomen, sowie gemeinsam für Alkandiyl mit 2 bis 6 Kohlenstoffatomen stehen,
sowie mit einem Chlorierungsmittel, gegebenenfalls in Gegenwart eines Verdünnungsmittels bei Temperaturen zwischen 20 und 120°C umsetzt.

2. Verfahren gemäß Anspruch 1, worin
R¹ für Wasserstoff, für Methyl, Ethyl sowie für gegebenenfalls einfach bis dreifach, gleich oder verschieden substituiertes Phenyl, wobei als Substituenten genannt seien: Fluor, Chlor, Methyl, Ethyl, Isopropyl, Methoxy, Methylthio, Trifluormethyl, Trifluormethoxy oder Trifluormethylthio, sowie gegebenenfalls durch Chlor und/oder Methyl substituiertes Phenyl steht und
R² und R³ gleich oder verschieden sind und für Methyl, Ethyl, n- oder i-Propyl, n-, i-, s- oder t-Butyl; Cyclopentyl oder Cyclohexyl; sowie gemeinsam für Butan-1,4-diyl oder Pentan-1,5-diyl stehen.

## Claims

1. Process for the preparation of chloromethylpyridines of the general formula (I) 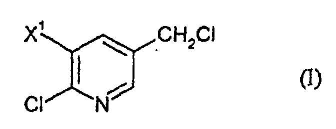 in which
X' represents hydrogen, chlorine or methyl,
**characterized in that**
pyridine derivatives of the general formula (II) 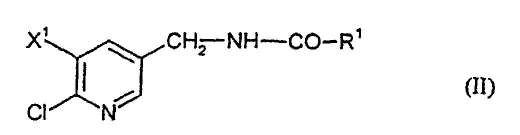 in which
R¹ represents hydrogen, or represents straight-chain or branched alkyl having 1 to 4 carbon atoms, or represents phenyl which is optionally mono- to trisubstituted by identical or different substituents, substituents which may be mentioned being: halogen, straight-chain or branched alkyl having 1 to 4 carbon atoms, straight-chain or branched alkoxy and alkylthio having in each case 1 to 2 carbon atoms, straight-chain or branched halogenoalkyl, halogenoalkoxy and halogenoalkylthio having in each case 1 to 2 carbon atoms and 1 to 5 identical or different halogen atoms, and phenyl which is optionally substituted by chlorine and/or methyl,
X¹ has the abovementioned meaning
are reacted by means of a formamide derivative of the general formula (III) 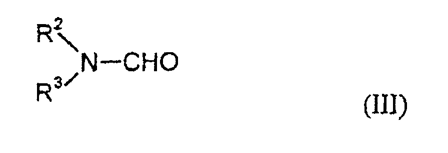 in which
R² and R³ are identical or different and represent straight-chain or branched alkyl having 1 to 6 carbon atoms, or furthermore represent cycloalkyl having 3 to 6 carbon atoms, or together represent alkanediyl having 2 to 6 carbon atoms,
and with a chlorinating agent, if appropriate in the presence of a diluent, at temperatures between 20 and 120°C.

2. Process according to Claim 1, wherein
R¹ represents hydrogen or represents methyl or ethyl or represents phenyl optionally substituted by one, two or three identical or different substituents selected from the group consisting of fluorine, chlorine, methyl, ethyl, isopropyl, methoxy, methylthio, trifluoromethyl, trifluoromethoxy and trifluoromethylthio, or represents optionally chlorine- and/or methyl-substituted phenyl, and
R² and R³ are identical or different and represent methyl, ethyl, n- or i-propyl, n-, i-, s- or t-butyl; cyclopentyl or cyclohexyl; or together represent 1,4-butanediyl or 1,5-pentanediyl.

## Revendications

1. Procédé de production de chlorométhylpyridines de formule générale (I) 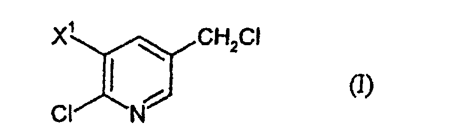 dans laquelle
X¹ est l'hydrogène, le chlore ou le groupe méthyle,
**caractérisé en ce qu'**on fait réagir des dérivés de pyridine de formule générale (II) 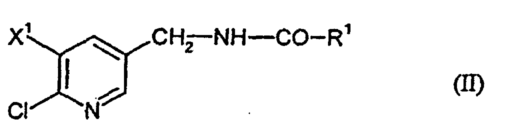 dans laquelle
R¹ représente l'hydrogène, un groupe alkyle linéaire ou ramifié ayant 1 à 4 atomes de carbone, ainsi qu'un groupe phényle portant éventuellement un à trois substituants, identiques ou différents, dont on peut mentionner les suivants : halogène, alkyle linéaire ou ramifié ayant 1 à 4 atomes de carbone, alkoxy et alkylthio linéaire ou ramifié ayant chacun 1 ou 2 atomes de carbone, halogénalkyle, halogénalkoxy et halogénalkylthio linéaire ou ramifié ayant chacun 1 ou 2 atomes de carbone et 1 à 5 atomes d'halogène identiques ou différents, ainsi que phényle éventuellement substitué par du chlore et/ou par un radical méthyle,
X¹ a la définition indiquée ci-dessus,
au moyen d'un dérivé de formamide de formule générale (III) 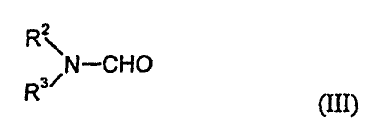 dans laquelle
R² et R³ sont identiques ou différents et représentent un groupe alkyle linéaire ou ramifié ayant 1 à 6 atomes de carbone, en outre un groupe cycloalkyle ayant 3 à 6 atomes de carbone, de même qu'ils peuvent former ensemble un groupe alcanediyle ayant 2 à 6 atomes de carbone,
ainsi qu'avec un agent de chloration, le cas échéant en présence d'un diluant, à des températures comprises entre 20 et 120 °C.

2. Procédé suivant la revendication 1, dans lequel
R¹ représente l'hydrogène, un groupe méthyle, éthyle ainsi qu'un groupe phényle portant éventuellement un à trois substituants identiques ou différents, dont on peut mentionner les suivants : fluor, chlore, méthyle, éthyle, isopropyle, méthoxy, méthylthio, trifluorométhyle, trifluorométhoxy ou trifluorométhylthio, ainsi que phényle éventuellement substitué par du chlore et/ou par un radical méthyle, et
R² et R³ sont identiques ou différents et représentent un groupe méthyle, éthyle, n-propyle, isopropyle, n-butyle, isobutyle, sec.-butyle, tertiobutyle ; cyclopentyle ou cyclohexyle ; de même qu'ils forment ensemble un groupe butane-1,4-diyle ou un groupe pentane-1,5-diyle.
